# EUROPEAN PATENT APPLICATION

(11) **EP 3 689 156 A1**
(43) Date of publication of application: **05.08.2020**
(21) Application number: 18861419.2
(22) Date of filing: 28.09.2018
(51) Int. Cl.: A23L 33/105, A61K 8/49, A61K 8/9789, A61Q 19/00

(54) **COMPOSITION FOR PROMOTING EXPRESSION OF AQUAPORIN 3**

(30) Priority: 29.09.2017 JP 2017191860
(71) Applicant: Suntory Holdings Limited, Osaka-shi, Osaka 530-8203 (JP)
(72) Inventor: NAKAMURA, Yumi, Kyoto 619-0284 (JP); FUKUI, Yuko, Kyoto 619-0284 (JP); TSUCHIYA, Takatsugu, Kyoto 619-0284 (JP); IZUMI, Reiko, Kyoto 619-0284 (JP)
(74) Representative: Vossius & Partner Patentanwälte Rechtsanwälte mbB
(86) International application number: PCT/JP2018/036448
(87) International publication number: WO 2019/066031

(57) **Abstract**

The purpose of the present invention is to provide a specific composition for promoting expression of aquaporin 3, and use thereof. The present invention relates to a composition for promoting expression of aquaporin 3, the composition including a galloyl group-containing flavan-3-ol monomer and/or a galloyl group-containing flavan-3-ol polymer as an active ingredient.

## Description

### TECHNICAL FIELD

The present invention relates to a composition for promoting expression of aquaporin 3, and use thereof.

### BACKGROUND ART

Many of women's skin-related problems and wishes regarding beauty are associated with skin moisturization and moisture richness, and studies are being actively conducted on techniques for improving skin moisture richness.

As a protein which can improve the skin moisture richness, aquaporin (AQP) is known.

AQP is a protein which was discovered in 1992 as a channel that allows a large amount of water to speedily pass therethrough, and it has been confirmed that a human has 13 types of AQPs (AQP0 to AQP12). These AQPs are differentially distributed depending on the types of cells and tissues. The AQPs are distributed in almost all of human organs, exist in a particularly large amount in organs involving active water transport, such as the kidney, act in a mutually coordinated manner, and perform a variety of functions including production of urine, secretion of digestive juice, protection against dryness, and binding of cells (Non Patent Literature 1).

The skin has a three-layer structure of epidermis, dermis and subcutaneous tissue in this order from the surface. The epidermis consists of a horny cell layer, a granulosa layer, a prickle cell layer and a basal layer. The horny cell layer functions to prevent evaporation of moisture into the air, and is capable of absorbing moisture from the ambient air and the inside of the skin, and of retaining the moisture. In the horny cell layer, there is no blood vessel serving as a water supply source. However, aquaporin 3 (AQP3) is expressed on cell membranes thereof, and absorbs water molecules from intercellular gaps to give passage to water, so that water can reach every part of the horny cell layer. In fact, the skin of a mouse lacking AQP3 is markedly lower in moisture content of the horny cell layer and skin elasticity than the skin of a wild-type mouse, and this indicates that AQP3 plays an important role in maintenance of skin moisture richness (Non Patent Literature 2). In addition, since AQP3 has a significant effect on the ability to recover from a wound such as skin roughness, it has been considered that enhancement of AQP3 production enables improvement of various skin troubles (Non Patent Literature 2).

It is known that AQP3 is expressed in skin epithelial cells. The following is also known: when the expression level of AQP3 increases, the water content of skin tissues increases, so that the skin appears rejuvenated, and skin elasticity is enhanced, so that the wound healing ability is improved (Non Patent Literature 3). On the other hand, it has been reported that a lack in expression of AQP3 leads to reduced elasticity and drying of skin tissues, and causes retardation of wound healing (Non Patent Literature 4) .

It is also considered that AQP3 functions as a glycerol transporter and can increase the amount of glycerol in the horny cell layer (Non Patent Literature 5).

### CITATION LIST

### - Patent Literature

Patent Literature 1: JP 2013-87058 A

### - Non Patent Literature

Non Patent Literature 1: Biology of Water and Aquaporin (2008), edited by Makoto Sasaki, Nakayama Shoten Co., Ltd.
Non Patent Literature 2: Roles of Aquaporin-3 in the Epidermis. Hara-Chikuma M. and Verkman AS, J Invest Dermatol(2008) 128:2145-2151
Non Patent Literature 3: Hydrating skin by stimulation biosynthesis of aquaporins. Dumas M., et al., J Drugs Dermatol.(2007) 6: s20-4
Non Patent Literature 4: Aquaporin-3 facilites epidermal cell migration and proliferation during wound healing. Hara-Chikuma M. and Verkman AS., J Mol Med.(2008)86:221-231
Non Patent Literature 5: Expression and function of aquaporins in human skin: Is aquaporin-3 justa glycerol transporter? Boury-Jamot M., et al., Biochim Biophys Acta.(2006)1758:1034-1042

### SUMMARY OF INVENTION

### - Technical Problem

For example, Patent Literature 1 discloses a preparation for enhancing aquaporin production which contains as an active ingredient an extract from a plant such as a grape leaf. However, an active ingredient which enhances production of aquaporin is not specified, and merely a plant extract as an active ingredient is disclosed. Here, the plant extract contains various ingredients, and when the plant extract is to be incorporated into a food or beverage product, a cosmetic, a pharmaceutical product, a quasi-pharmaceutical product or the like, it is desirable to specify an ingredient which enhances production of targeted aquaporin.

The present invention has been made for solving the above-described problems, and an object of the present invention is to provide a specific composition for promoting expression of aquaporin 3, and use thereof.

### - Solution to Problem

The present inventors have extensively conducted studies, and resultantly found that a specific grape-derived material as described later can improve skin moisture richness. Further, the present inventors have found that the later-described specific grape-derived material can increase the expression level of aquaporin 3 in skin epithelial cells and that among ingredients present in the grape-derived material, a galloyl group-containing flavan-3-ol monomer and a galloyl group-containing flavan-3-ol polymer (oligomeric proanthocyanidin (OPC)) can particularly increase the expression level of aquaporin 3. Thus, the present invention has been completed.

That is, the present invention relates to the following (1) to (9).
(1) A composition for promoting expression of aquaporin 3, including a galloyl group-containing flavan-3-ol monomer and/or a galloyl group-containing flavan-3-ol polymer as an active ingredient.
(2) The composition according to (1), wherein the galloyl group-containing flavan-3-ol monomer and/or the galloyl group-containing flavan-3-ol polymer is present in a grape-derived material.
(3) The composition according to (2), wherein the grape-derived material contains at least one selected from the group consisting of at least one raw material selected from the group consisting of a pericarp, a fruit or a seed of grape; a fermented product of the raw material; an extract thereof; or a processed product thereof.
(4) The composition according to any one of (1) to (3), wherein the galloyl group-containing flavan-3-ol monomer is a compound represented by the following formula (1) and/or a compound represented by the following formula (2) . 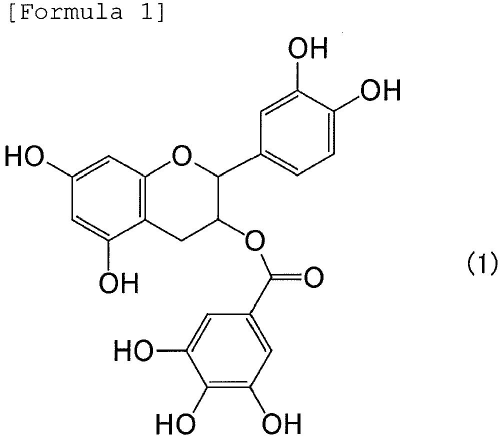 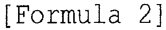 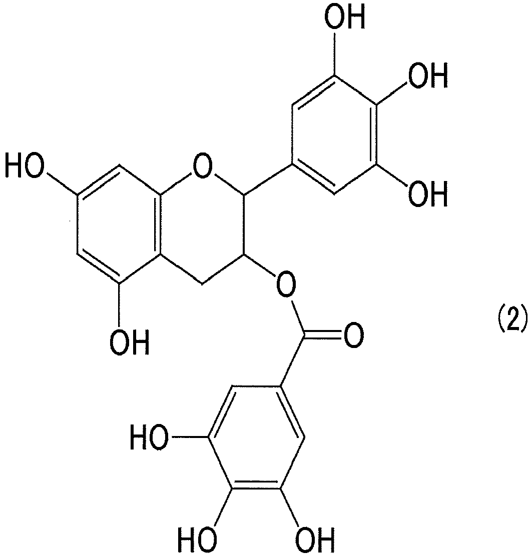
(5) The composition according to any one of (1) to (4), wherein the galloyl group-containing flavan-3-ol polymer is a compound represented by the following formula (3) and/or a compound represented by the following formula (4) . 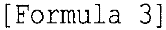 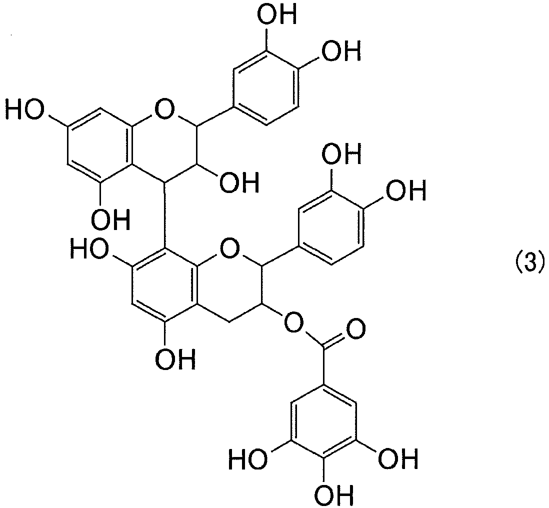 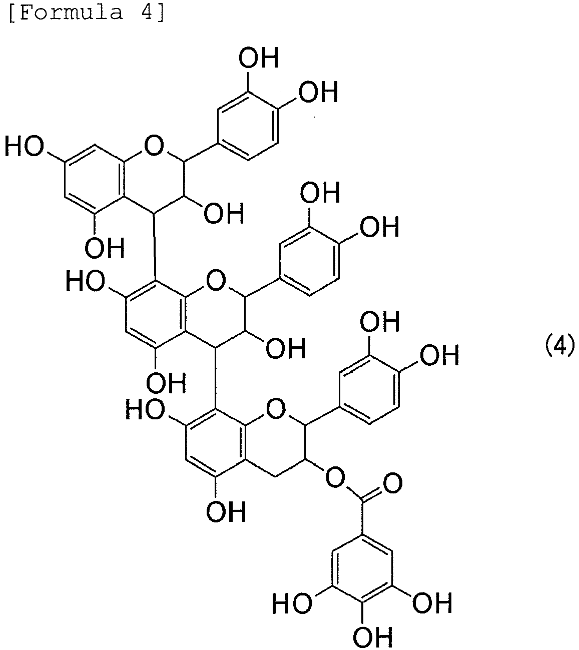
(6) The composition according to any one of (1) to (5), wherein the composition is a food or beverage product, a cosmetic, a pharmaceutical product or a quasi-pharmaceutical product.
(7) The composition according to any one of (1) to (6), wherein the composition is used for one or more selected from the group consisting of improvement of skin moisture richness, supply of moisture to the skin, retention of skin moisture, skin moisturization, relief of skin dryness, improvement of skin condition, enhancement of skin barrier functions, prevention of skin roughness, improvement of skin roughness, retention of skin functions, improvement of skin functions or promotion of wound healing.
(8) Use of a galloyl group-containing flavan-3-ol monomer and/or a galloyl group-containing flavan-3-ol polymer for promoting expression of aquaporin 3.
(9) Use of a galloyl group-containing flavan-3-ol monomer and/or a galloyl group-containing flavan-3-ol polymer for improving skin moisture richness by promoting expression of aquaporin 3.

### - Advantageous Effects of Invention

A galloyl group-containing flavan-3-ol monomer and/or a galloyl group-containing flavan-3-ol polymer can promote expression of aquaporin 3. Therefore, the composition of the present invention including a galloyl group-containing flavan-3-ol monomer and/or a galloyl group-containing flavan-3-ol polymer as an active ingredient can increase the expression level of aquaporin 3. Further, the composition of the present invention including a galloyl group-containing flavan-3-ol monomer and/or a galloyl group-containing flavan-3-ol polymer as an active ingredient can improve skin moisture richness by promoting expression of aquaporin 3.

### BRIEF DESCRIPTION OF DRAWINGS

FIG. 1(a) is a graph showing the water contents of horny cell layers of subjects in an evaluation test for skin moisture richness; and FIG. 1(b) is a graph showing the amounts of change of the water contents of horny cell layers of subjects in the evaluation test for skin moisture richness.
FIG. 2 is a graph showing the results of measuring the expression levels of aquaporin 3 when the compositions of Examples 2-1 to 2-8 in the present invention were used.
FIG. 3 is a graph showing the results of measuring the expression levels of aquaporin 3 when the fraction of the composition of Example 2-6 was used.
FIG. 4 is a graph showing the results of measuring the expression levels of aquaporin 3 when the compositions of Examples 3-1 to 3-4 in the present invention were used.
FIG. 5 is a graph showing the results of measuring the expression levels of aquaporin 3 when the compositions of Examples 4-1 to 4-4 in the present invention were used.
FIG. 6 is a graph showing the results of measuring the expression levels of aquaporin 3 when the compositions of Examples 5-1 and 5-2 in the present invention were used.

### DESCRIPTION OF EMBODIMENTS

Hereinafter, the present invention will be described in detail. The following embodiments give illustrative examples for understanding the present invention, and are not intended to limit the present invention to these embodiments. The present invention can be carried out in various modes without departing from the spirit of the present invention.

The composition of the present invention includes a galloyl group-containing flavan-3-ol monomer and/or a galloyl group-containing flavan-3-ol polymer as an active ingredient, and is used for "promoting expression of aquaporin 3" or "improving skin moisture richness by promoting expression of aquaporin 3".

The composition for improving skin moisture richness is referred to as the composition according to a first embodiment of the present invention. The composition for promoting expression of aquaporin 3 is referred to as the composition according to a second embodiment of the present invention.

Hereinafter, features etc. common to the composition according to the first embodiment and the composition according to the second embodiment of the present invention will be described. In the following description, the composition according to the first embodiment and the composition according to the second embodiment of the present invention are also referred to collectively as "the composition according to the present invention".

The galloyl group-containing flavan-3-ol monomer is a compound in which a galloyl group is bonded to flavan-3-ol typified by catechin, epicatechin or the like, and the bonding position of the galloyl group is not limited. For example, the galloyl group may be bonded to a flavan backbone. In one aspect, the galloyl group-containing flavan-3-ol monomer in the present invention may be a compound in which a galloyl group is bonded to a hydroxyl group at position 3 on the flavan backbone, that is, a compound in which a hydroxyl group at position 3 on the flavan backbone forms an ester bond with gallic acid.

Examples of the compound in which a galloyl group is bonded to a hydroxyl group at position 3 on flavan-3-ol include compounds represented by the above formula (1) and compounds represented by the above formula (2).

Specific examples thereof include catechin gallate, epicatechin gallate, epigallocatechin gallate and gallocatechin gallate. Among them, epicatechin gallate is preferable.

The flavan-3-ol polymer is a dimeric or higher order polymer in which flavan-3-ol as a constituent unit is bonded at position 4-6 or 4-8. The flavan-3-ol polymer is a type of polyphenol, and this compound is also referred to condensed tannin.

The galloyl group-containing flavan-3-ol polymer is a flavan-3-ol polymer including galloyl group-modified flavan-3-ol as at least one constituent unit. The bonding position of the galloyl group in flavan-3-ol as a constituent unit is not limited, and for example, the galloyl group-containing flavan-3-ol polymer in the present invention is preferably flavan-3-ol polymer having, as a constituent unit, flavan-3-ol in which a galloyl group is bonded at position 3 on a flavan backbone (flavan-3-ol in which a hydroxyl group at position 3 on the flavan backbone forms an ester bond with gallic acid).

The galloyl group-containing flavan-3-ol polymer may be a mixture of two or more polymers different in polymerization degree etc.

The flavan-3-ol polymer is a dimeric or higher order polymer in which flavan-3-ol as a constituent unit is bonded at position 4-8. For example, as compared to a flavan-3-ol dimer in which flavan-3-ol is bonded at position 4-6, a flavan-3-ol dimer in which flavan-3-ol is bonded at position 4-8 is contained in a larger amount in natural products such as grape, so that it is possible to acquire a more sufficient amount of the flavan-3-ol dimer for promoting expression of aquaporin 3.

Examples of the flavan-3-ol polymer having, as a constituent unit, flavan-3-ol in which a galloyl group is bonded to a hydroxyl group at position 3 on a flavan backbone include compounds represented by the above formula (3) and compounds represented by the above formula (4).

The galloyl group-containing flavan-3-ol monomer and/or the galloyl group-containing flavan-3-ol polymer for use in the present invention is a composition to be applied to a food or beverage product, a cosmetic, a pharmaceutical product or a quasi-pharmaceutical product, and hence is preferably monomeric to trimeric. The galloyl group-containing flavan-3-ol monomer and/or the galloyl group-containing flavan-3-ol polymer is more preferably dimeric and/or trimeric, still more preferably dimeric. A flavan-3-ol monomer and a dimeric flavan-3-ol polymer can be absorbed into the body when orally ingested. As a composition to be used for external preparations, a flavan-3-ol monomer and a dimeric and/or trimeric flavan-3-ol polymer are preferable in view of absorption and penetration into keratinocytes at which aquaporin 3 is expressed.

Flavan-3-ol monomers such as catechin and epicatechin and dimeric and/or trimeric flavan-3-ol polymers are low in action of "promoting expression of aquaporin 3" or "improving skin moisture richness by promoting expression of aquaporin 3"; however, presence of a galloyl group improves the action. In particular, in dimeric and/or trimeric flavan-3-ol polymers, presence of a galloyl group remarkably improves the action. Thus, at least one selected from the group consisting of a galloyl group-containing flavan-3-ol monomer or dimeric or trimeric galloyl group-containing flavan-3-ol polymers can be circulated in blood and absorbed into keratinocytes, and can provide a composition which is high in the above-described action. Further, in view of aquaporin 3 expression promoting action, it is preferable that the composition according to the present invention include a galloyl group-containing flavan-3-ol polymer as an active ingredient.

The galloyl group-containing flavan-3-ol polymer to be used for the composition according to the present invention is not limited, and preferred examples thereof include galloyl group-containing flavan-3-ol dimers such as (+)-catechin-(-)epicatechin-3-0-gallate (hereinafter, also referred to as C-ECG) and (-)-epicatechin-(-)-epicatechin-3-0-gallate (hereinafter, also referred to as EC-ECG), and galloyl group-containing flavan-3-ol trimers such as (+)-catechin-(+)-catechin-(-)-epicatechin-3-O-gallate (hereinafter, also referred to as C-C-ECG).

The galloyl group-containing flavan-3-ol monomer and/or the galloyl group-containing flavan-3-ol polymer is not limited by the origin or production method thereof. For example, a plant-derived compound extracted from a plant, or a compound obtained by synthesis may be used. The galloyl group-containing flavan-3-ol monomer can be obtained from plants such as grape and tea plants. The galloyl group-containing flavan-3-ol polymer can be obtained from plants such as grape, European pear and lichee. For example, grape seeds include a galloyl group-containing flavan-3-ol monomer and a galloyl group-containing flavan-3-ol polymer (Reference 1: "Comparative flavan-3-ol composition of seeds from different grape varieties" Food Chemistry 53 (1995) 197-201; Reference 2: "PROCYANIDIN DIMERS AND TRIMERS FROM GRAPE SEEDS" Phytochemistry Vol. 30. No. 4, pp 1259-1264 1991; Reference 3: "Application of 'Grape Seed Polyphenol' Functionality" NEW FOOD INDUSTRY Vol. 43 No. 11 (2001), Separate Volume).

In view of improving skin moisture richness and increasing the expression level of aquaporin 3, the galloyl group-containing flavan-3-ol and/or galloyl group-containing flavan-3-ol polymer is preferably one present in a grape-derived material. A galloyl group-containing flavan-3-ol monomer and a galloyl group-containing flavan-3-ol polymer which are present in a grape-derived material are suitable in view of safety.

The grape-derived material contains at least one selected from the group consisting of at least one raw material selected from the group consisting of a pericarp, a fruit or a seed of grape; a fermented product of the raw material; an extract thereof; or a processed product thereof. Preferably, the grape-derived material contains at least one selected from the group consisting of a pericarp and/or a seed of grape, a fermented product of a pericarp and/or a seed of grape, an extract thereof or a processed product thereof. More preferably, the grape-derived material contains a pericarp and/or a seed of grape as a grape raw material.

Preferably, the composition according to the present invention contains the grape-derived material including a galloyl group-containing flavan-3-ol monomer and/or a galloyl group-containing flavan-3-ol polymer.

As used herein, the "extract of a raw material" means some ingredients obtained from a raw material.

Examples of the extract of a raw material include fruit juices (straight juice and concentrated juice).

Examples of the fermented product of a raw material include wines.

Examples of the extract of a fermented product of a raw material include an extracted product from wine fermentation residues with a solvent such as water or ethanol.

Examples of the processed product of a fermented product of a raw material include concentrated wines, diluted wines, and wines in which specific ingredients are concentrated or removed. Wines from which alcohol components distilled away, and the like are preferable.

The extract of a raw material can be obtained through a common extraction method that is used for extraction of plant ingredients. The extraction method can be appropriately set, and the extraction conditions are not limited. For example, the at least one raw material selected from the group consisting of a pericarp, a fruit or a seed of grape may be extracted with a solvent at normal temperature, high temperature or low temperature.

The raw material may be ground when extraction is performed.

Examples of the method for grinding the raw material include a method in which a raw material is ground in liquid nitrogen as described in JP 4892348 B.

For obtaining the extract of a raw material in the form of an extract liquid, the extract of the raw material may be obtained by adding the raw material to a solvent, and optionally performing heating, stirring or the like to extract ingredients.

The solvent to be used for extraction (extraction solvent) is preferably water, an organic solvent or a mixture thereof. Examples of the organic solvent include ethanol, 1,3-butylene glycol (labeling designation: BG), 1,2-pentanediol (labeling designation: pentanediol), propane-1,2-diol (labeling designation: propylene glycol), 1,3-propanediol (labeling designation: pentanediol) and squalane. One organic solvent may be used singly, or two or more organic solvents may be combined.

In extraction, an acid or an alkali may be added to adjust the pH of the extraction solvent.

In extraction, pressure may be optionally applied. The extraction temperature is not limited, and is preferably 0°C to 130°C, more preferably 20°C to 100°C, still more preferably 50°C to 100°C, furthermore preferably 70 to 95°C, for example. The extraction temperature is preferably not higher than the boiling point of the solvent.

The extraction time can be appropriately set according to extraction conditions such as an extraction temperature.

It is preferable to remove extraction residues from the resulting extract after extraction. The method for removing the extraction residues is not limited, and a known method such as filtration or centrifugation can be employed.

In the present invention, an extract liquid obtained through extraction may be used as a grape-derived material. The extract liquid may be diluted with an appropriate solvent to obtain a grape-derived material, and the extract liquid may be subjected to a concentrating or drying step to obtain a concentrate or a dried product. The concentrating or drying method is not limited, and a known method such as freeze-drying or spray drying can be used.

The form of the extract is not limited, and the extract can be used in the form of liquid, powder or paste. As long as the effect of the present invention is not impaired, the extract can be purified or separated through an appropriate purification method or separation method (e.g. liquid-liquid extraction (liquid-liquid partition) or chromatography) to obtain fractions having high activity.

The grape-derived materials in the present invention include various solvent-extracted liquids obtained through the above-described extraction methods, diluted liquids thereof, concentrates thereof, dried products thereof, and purified fractions thereof.

The fermented product of a raw material is not limited, and for example, a fermented product obtained with yeast can be used. As yeast, for example, *Saccharomyces cerevisiae* can be used. It is preferable to appropriately determine fermentation conditions according to the type of yeast.

In the composition according to the present invention, the species of grape used as an origin of the grape-derived material is not limited, and is preferably a species classified as a material for wine. Examples of the species classified as a material for wide include species classified as a material for red wine and species classified as a material for white wine.

The species classified as a material for red wine is preferably at least one selected from the group consisting of *Merlot, Garnacha Tintorera, Ancellotta, Monastrell, Tempranillo, Cabernet Sauvignon, Pinot Noir, Muscat Bailey* A or *Concord.*

Examples of the species classified as a material for white wine, which can be used, include, but are not limited to, *Chardonnay, Chablis* and *Koshu.*

The grape-derived materials from these species include a galloyl-containing flavan-3-ol monomer and a galloyl-containing flavan-3-ol polymer, and therefore exhibit an excellent improving effect on skin moisture richness and an excellent increasing effect on the expression level of aquaporin 3.

Preferably, the galloyl-containing flavan-3-ol monomer and/or the galloyl-containing flavan-3-ol polymer as an active ingredient of the composition according to the present invention is obtained from the above-described grape-derived materials. Examples of the method for obtaining a galloyl-containing flavan-3-ol monomer and/or a galloyl-containing flavan-3-ol polymer from a grape-derived material include a separation method, and it is possible to use a method that is commonly used in the technical field of the present invention.

For example, in extraction and purification of a galloyl-containing flavan-3-ol monomer and/or a galloyl-containing flavan-3-ol polymer from grape seeds, a grape seed extract including a galloyl-containing flavan-3-ol monomer and/or a galloyl-containing flavan-3-ol polymer can be obtained by extracting grape seeds with a hydrous alcohol, then filtering and concentrating the resulting extract liquid to remove the alcohol, and then performing purification such as column purification. The purity of the galloyl-containing flavan-3-ol monomer and/or the galloyl-containing flavan-3-ol polymer can be enhanced by further purifying the resulting grape seed extract.

The galloyl group-containing flavan-3-ol monomer can be obtained from a tea plant-derived material besides grape. For example, the galley group-containing flavan-3-ol monomer can be extracted from a green tea leaf, and purified.

For example, generation of gallic acid at the time of subjecting a flavan-3-ol monomer and polymer to tannase treatment or acid hydrolysis treatment indicates the presence of a galloyl group in the flavan-3-ol monomer and polymer. A flavan-3-ol monomer and polymer in which gallic acid is generated by the treatment contain a galloyl group.

Generation of gallic acid can be confirmed by measuring the amounts of gallic acid in the flavan-3-ol monomer and polymer before and after the tannase treatment or the acid hydrolysis treatment. When the amount of gallic acid in the flavan-3-ol monomer and polymer after the treatment is larger than the amount of gallic acid in the flavan-3-ol monomer and polymer before the treatment, it can be considered that gallic acid has been generated by the treatment.

The composition according to the present invention is preferably a food or beverage product, a cosmetic, a pharmaceutical product or a quasi-pharmaceutical product. The method for producing such a food or beverage product, cosmetic, pharmaceutical product or quasi-pharmaceutical product is not limited, and the active ingredient in the present invention or the grape-derived material containing the active ingredient may be blended in such a product. The composition according to the present invention may be a material composition to be used as a material for the food or beverage product, cosmetic, pharmaceutical product or quasi-pharmaceutical product.

The composition according to the present invention may be used for one or more selected from the group consisting of improvement of skin moisture richness, retention of skin moisture, supply of moisture to the skin, skin moisturization, relief of skin dryness, enhancement of skin barrier functions, improvement of skin condition, prevention of skin roughness, improvement of skin roughness, retention of skin functions, improvement of skin functions or promotion of wound healing.

As used herein, the "food or beverage product" is a generic term of orally ingestible materials which include solid, fluent and liquid materials and mixtures thereof.

Examples of the food or beverage product include food for specified health use, nutritional supplements, health food, food with function claims, infant food, infant modified milk, premature infant modified milk and food for the elderly.

The food for specified health use means food with an indication that persons who ingest the food for a specific health purpose in the food life can expect to satisfy the health purpose by ingestion of the food under approval referred to in Article 26, paragraph (1) or permission referred to in Article 29, paragraph (1) of the Health Promotion Act.

The nutritional supplement refers to food enriched with specific nutritional ingredients. The health food refers to healthy food or food beneficial to health, and includes nutritional supplements, natural food and diet food.

The food with function claims refers to food registered to Consumer Affairs Agency as food with functional claims based on scientific grounds.

The infant food refers to food to be given to children aged about 6 or younger.

The infant modified milk refers to modified milk to be given to children aged about 1 or younger.

The premature infant modified milk refers to modified milk to be given to premature infants until about the sixth month after birth.

The food for the elderly refers to food treated so as to be digested and absorbed more easily as compared to untreated food.

The form of the food or beverage product is not limited, and various forms can be applied. Examples of such forms include beverages, confectionery and supplements.

The beverage may be either an alcoholic beverage or a non-alcoholic beverage.

Examples of the non-alcoholic beverage include, but are not limited to, tea-based beverages, coffee beverages, carbonated beverages, functional beverages, fruit/vegetable-based beverages, milk beverages, soymilk beverages and flavor water. Further, the non-alcoholic beverages include beverages having an alcohol content of less than 1%, and examples thereof include non-alcoholic beers, non-alcoholic wines and non-alcoholic cocktails.

Examples of the tea-based beverage include black tea beverages and sugar-free tea beverages.

Examples of the sugar-free tea beverage include green tea beverages, oolong tea beverages, barley tea beverages, brown rice tea beverages, adlay tea beverages and sugar-free black tea beverages.

The coffee beverage may be either packaged coffee or liquid coffee.

Examples of the carbonated beverage include cola-flavored beverages, transparent carbonated beverages, ginger ales, fruit juice-based carbonated beverages, carbonated beverages with milk and sugar-free carbonated beverages.

Examples of the functional beverage include beauty drinks, sports drinks, energy drinks, health supporting beverages and pouched jerry beverages.

Examples of the fruit/vegetable-based beverage include fruit beverages, beverages with fruit, soft drinks with a low content of fruit juice, pulp-containing fruit beverages and fruity flesh beverages.

Examples of the milk beverage include bovine milk, yogurt drinks, lactic acid bacteria beverages and soft drinks with milk.

Examples of the soymilk beverage include soymilk and soybean beverages.

The form in which a beverage is stored is not limited, and may be a packaged beverage. The container for the packaged beverage is not limited, and a container of any form and material may be used. It is possible to use any of containers that are commonly used, such as metallic containers such as aluminum cans and steel cans; resin containers such as plastic bottles; paper containers such as paper packs; glass containers such as glass bottles; and wood containers such as barrels. Such a container is filled with a beverage, and hermetically sealed to obtain a packaged beverage.

The packaged beverage according to the present invention is not limited to one in which the beverage is drunk directly from the container. For example, a beverage packed in a bulk container such as a bag-in box, or a portion container can be poured into another container when served as a drink. A concentrated liquid can be diluted when served as a drink.

Examples of the alcoholic beverage include beers, beer-based beverages, wines, distilled spirits, distilled spirits diluted with soda or juice, liqueurs, cocktails, spirits, whiskeys and brandies.

Examples of the beer-based beverage include low-malt beers or third beers, and beers with fruit or fruit juice.

When the composition according to the present invention is used as a food or beverage product, the food or beverage product may contain sweeteners such as stevia extract, acesulfame potassium and sucralose; sugars such as sucrose; organic acids such as malic acid and citric anhydride; dietary fibers such as hardly digestible dextrin; flavors; and colorants.

When the composition according to the present invention is a food or beverage product, it may be a skincare food or beverage product aimed at keeping the skin healthy and conditioning the skin itself. Examples of the form of such a food or beverage product include beverages and supplements.

When used as a cosmetic, the composition according to the present invention is preferably used as a skin external composition.

When used as the skin external composition, the composition according to the present invention can be prepared as a skincare cosmetic such as a tape, an ointment, a face wash (face washing cosmetic), a beauty lotion, a pack, a massage cream, a milky lotion, an emulsion, a cream or an essence (serum); or a body-care cosmetic such as a soap, a liquid cleanser, a bath salt, a sun-block cream, a tanning oil, a deodorant spray, a body lotion, a body cream or a hand cream.

The cosmetic may further contain a carrier and an excipient which can be used for cosmetics.

When the composition according to the present invention is used as a pharmaceutical product or a quasi-pharmaceutical product, the route of administration of the pharmaceutical product or the quasi-pharmaceutical product may be either oral or parenteral.

Examples of the parenteral administration include transdermal administration, topical administration, transnasal administration, sublingual administration, pulmonary administration, enteral administration, transmucosal administration and injection.

Examples of the dosage form of a preparation for oral administration include solutions, tablets, powders, pills, subtle granules, granules, sugar-coated tablets, capsules, suspensions, emulsion formulations, syrups, extracts, emulsions, microcapsules, troches, lozenges, buccals and chewables.

Examples of the dosage form of a preparation for parenteral administration include aerosols, injections, drops, inhalations, transfusions, suppositories, transdermal absorbents, nasal drops, eye drops, cataplasms, plasters, creams, gels and lotions.

When the composition according to the present invention is used as a pharmaceutical product or a quasi-pharmaceutical product, the composition according to the present invention may be used singly, or in combination with another pharmaceutical product or quasi-pharmaceutical product.

When the composition according to the present invention is used as a pharmaceutical product or a quasi-pharmaceutical product, the pharmaceutical product or the quasi-pharmaceutical product may contain one or more additive selected from the group consisting of a pharmaceutically acceptable carrier, an excipient, a buffer, a binder, a disintegrant, a lubricant, an antioxidant or a colorant.

The composition according to the present invention may be used in the form of a preparation, for example, and the present invention is not limited thereto. The preparation as is may be used as the composition, or the composition may contain the preparation.

As described above, the composition according to the present invention includes a galloyl group-containing flavan-3-ol monomer and/or a galloyl group-containing flavan-3-ol polymer as an active ingredient. It is preferable that the galloyl group-containing flavan-3-ol monomer and/or the galloyl group-containing flavan-3-ol polymer be present in a grape-derived material. In one aspect, it is more preferable that the composition according to the present invention contain a grape-derived material including a galloyl group-containing flavan-3-ol monomer and/or a galloyl group-containing flavan-3-ol polymer.

The content (in terms of a dried product) of the grape-derived material in the composition according to the present invention is preferably 1 wt% to 30 wt%.

It is more preferable that the composition according to the present invention include as an active ingredient a galloyl group-containing flavan-3-ol polymer present in a grape-derived material.

Hereinafter, the flavan-3-ol polymer present in a grape-derived material is abbreviated as "grape-derived OPC", and the galloyl group-containing flavan-3-ol polymer present in a grape-derived material is abbreviated as "galloyl group-containing grape-derived OPC".

When the composition according to the present invention is a material composition to be used as a material for a food or beverage product, a cosmetic, a pharmaceutical product, a quasi-pharmaceutical product or the like, the concentration of grape-derived OPC present in the material composition may be 100 ppm or more, 200 ppm or more, 500 ppm or more, 1,000 ppm or more or 2,000 ppm or more. The concentration of grape-derived OPC present in the material composition is preferably 50,000 ppm or less, more preferably 25,000 ppm or less, still more preferably 20,000 or less. The concentration of grape-derived OPC present in the material composition is preferably 100 ppm to 50,000 ppm, more preferably 200 ppm to 25,000 ppm, still more preferably 500 ppm to 20,000 ppm, for example. In another aspect, the concentration of grape-derived OPC present in the material composition is preferably 500 ppm to 50,000 ppm, more preferably 1,000 ppm to 25,000 ppm, still more preferably 2,000 ppm to 20,000 ppm. The concentration of galloyl group-containing grape-derived OPC present in the material composition may be 1.0 ppm or more, 2.0 ppm or more, 5.0 ppm or more, 10.0 ppm or more or 20.0 ppm or more. The concentration of galloyl group-containing grape-derived OPC present in the material composition is preferably 5,000 ppm or less, more preferably 2,500 ppm or less, still more preferably 2,000 or less. The concentration of galloyl group-containing grape-derived OPC present in the material composition is preferably 1.0 ppm to 5,000 ppm, more preferably 2.0 ppm to 2,500 ppm, still more preferably 5.0 ppm to 2,000 ppm, for example. In another aspect, the concentration of galloyl group-containing grape-derived OPC present in the material composition is preferably 5.0 ppm to 5,000 ppm, more preferably 10.0 ppm to 2,500 ppm, still more preferably 20.0 ppm to 2,000 ppm.

When the composition according to the present invention is a material for a food or beverage product, a cosmetic, a pharmaceutical product or a quasi-pharmaceutical product, the concentration of grape-derived OPC present in the composition according to the present invention is preferably 0.001 mg/mL or more, more preferably 0.01 mg/mL or more, still more preferably 0.1 mg/mL or more. The concentration of grape-derived OPC present in the composition according to the present invention is preferably 100.0 mg/mL or less, more preferably 10.0 mg/mL or less. The concentration of grape-derived OPC present in the composition according to the present invention is preferably 0.001 mg/mL to 100.0 mg/mL, more preferably 0.01 mg/mL to 10.0 mg/mL in the composition. In another aspect, the concentration of grape-derived OPC present in the composition according to the present invention is preferably 0.01 mg/mL to 100.0 mg/mL, more preferably 0.1 mg/mL to 10.0 mg/mL. The concentration of galloyl group-containing grape-derived OPC present in the composition according to the present invention is preferably 0.00001 mg/mL or more, more preferably 0.0001 mg/mL or more, still more preferably 0.001 mg/mL or more. The concentration of galloyl group-containing grape-derived OPC present in the composition according to the present invention is preferably 10.0 mg/mL or less, more preferably 1.0 mg/mL or less. The concentration of galloyl group-containing grape-derived OPC present in the composition according to the present invention is preferably 0.00001 mg/mL to 10.0 mg/mL, more preferably 0.0001 mg/mL to 1.0 mg/mL. In another aspect, the concentration of galloyl group-containing grape-derived OPC present in the composition according to the present invention is preferably 0.0001 mg/mL to 10.0 mg/mL, more preferably 0.001 mg/mL to 1.0 mg/mL. The concentration of grape-derived OPC can be measured through the method described in JP 4659407 B. This amount of the composition may be used at a time, or used in several parts. The content of galloyl group-containing grape-derived OPC can be measured in accordance with a known method, for example a LC-MS method.

When the composition according to the present invention is orally ingested, the amount of grape-derived OPC ingested per day is preferably 10 mg to 2,000 mg, more preferably 100 mg to 400 mg. Here, the amount of galloyl group-containing grape-derived OPC ingested per day is preferably 0.1 mg to 200 mg, more preferably 1.0 mg to 40.0 mg.

When the composition according to the present invention is used as an external agent such as a skin external composition, the amount (in terms of a dried product) of grape-derived OPC used per day is preferably 0.01 mg to 100.0 mg, more preferably 0.1 mg to 10.0 mg. Here, the amount (in terms of a dried product) of galloyl group-containing grape-derived OPC used per day is preferably 0.0001 mg to 10.0 mg, more preferably 0.001 mg to 1.0 mg.

The above-described amount of the composition may be used at a time, or used in several parts.

The composition according to the present invention is suitably used for humans. In one aspect, the composition according to the present invention is suitably used for subjects desiring or needing an increase in expression of aquaporin 3 or subjects desiring or needing improvement of skin moisture richness.

The composition according to the present invention may contain grape-derived anthocyanin.

In the composition according to the present invention, the content of total polyphenol derived from grape content is preferably 500 mg/mL to 50,000 mg/mL, more preferably 1,000 mg/mL to 20,000 mg/mL.

The content of total polyphenol derived from grape can be measured through the Folin-Ciocalteu method.

The composition including a galloyl group-containing flavan-3-ol monomer and/or a galloyl group-containing flavan-3-ol polymer according to the present invention can be produced through one of methods (1) to (3) below.
(1) Wine containing a large amount of polyphenol is adjusted to an appropriate concentration to give an alcohol-containing wine-like beverage, which is taken as a composition including a galloyl group-containing flavan-3-ol monomer and/or a galloyl group-containing flavan-3-ol polymer.
(2) Wine containing a large amount of polyphenol is concentrated under reduced pressure, so that ethanol is distilled away to produce a non-alcoholic processed product, and this product is adjusted to an appropriate concentration to give a non-alcoholic beverage, which is taken as a composition including a galloyl group-containing flavan-3-ol monomer and/or a galloyl group-containing flavan-3-ol polymer.
(3) A grape raw material is ground (for example, ground in liquid nitrogen), the ground product is extracted with an aqueous solution containing ethanol in an amount of 0% to 100%, and the resulting extract is adjusted to an appropriate concentration to give a beverage, which is taken as a composition including a galloyl group-containing flavan-3-ol monomer and/or a galloyl group-containing flavan-3-ol polymer.

The composition produced through one of the above-described methods includes various ingredients in addition to a galloyl group-containing flavan-3-ol monomer and/or a galloyl group-containing flavan-3-ol polymer which is an active ingredient. Thus, by further subjecting the resulting composition to separation etc., a composition having a high content of a grape-derived galloyl group-containing flavan-3-ol monomer and/or galloyl group-containing flavan-3-ol polymer can be produced.

### (First Embodiment)

A composition according to the first embodiment of the present invention, which is a skin moisture richness improving composition, will now be described. The composition includes a galloyl group-containing flavan-3-ol monomer and/or a galloyl group-containing flavan-3-ol polymer as an active ingredient. The composition improves skin moisture richness by increasing expression of aquaporin 3.

The method for using the composition according to the first embodiment of the present invention is not limited, and the composition may be absorbed into the body by oral ingestion, or applied to the skin by putting it on or the like.

Among these, the method in which the composition is absorbed into the body by oral ingestion is preferable.

As used herein, the term "skin moisture richness" refers to a state in which moisture of the horny cell layer is retained.

As used herein, the term "improving skin moisture richness" means that reduction of the moisture content of the horny cell layer is prevented, a state in which moisture of the horny cell layer is moderately retained is maintained, or the water content of the horny cell layer is increased by increasing the moisture content of the horny cell layer, preventing evaporation of skin moisture, and/or increasing absorption of moisture into the horny cell layer.

It is preferable that the composition according to the first embodiment of the present invention be used for increasing the water content of the horny cell layer, preventing evaporation of skin moisture or increasing absorption of moisture into the horny cell layer by increasing expression of aquaporin 3.

Use of the composition according to the first embodiment of the present invention enables improvement of user's skin moisture richness.

An example of the method for measuring the water content of the horny cell layer will now be described. The following example shows a method for measuring the water content of a part of a face. The method for measuring the water content of the horny cell layer is not limited to the following example, and for example, the water content in the horny cell layer of an arm may be measured.

### (Method for Measuring Water Content of Horny Cell Layer)

A subject who has finished cleansing away a cosmetic and washing the face is guided into a constant temperature and humidity room (temperature: 21±1°C, humidity 50±5%), and acclimatized at rest for 20 minutes.

Next, a portion which is a point of intersection between the tail of the eye and the nostril is designated as a measurement portion, and measurements are made at the same position. In the measurement, the water content of the horny cell layer at the above-described portion (the same position) is measured five times with Corneometer CM825 (manufactured by Courage + Khazaka electronic GmbH).

Among the obtained values, the highest value and the lowest value are excluded, and an average of the other three measured values is defined as the "water content of the horny cell layer".

The composition according to the first embodiment of the present invention may be used for one or more selected from the group consisting of improvement of skin moisture richness, skin moisturization, relief of skin dryness, enhancement of skin barrier functions, improvement of skin condition, prevention of skin roughness, improvement of skin roughness, retention of skin functions, improvement of skin functions or promotion of wound healing.

These are effects produced as a result of increasing the water content of the horny cell layer.

One factor of the increasing effect on the water content of the horny cell layer may be that the galloyl group-containing flavan-3-ol monomer and/or the galloyl group-containing flavan-3-ol polymer present in the composition of the present invention increases the expression level of aquaporin 3 in skin epithelial cells.

It has been reported that aquaporin 3 is a water transporter, and that when the expression level of aquaporin 3 in skin epithelial cells is increased, absorption of water into the horny cell layer increases, leading to an increase in water content of the horny cell layer (Non-Patent Literature 2).

It is considered that aquaporin 3 can function as a glycerol transporter to increase the glycerol content of the horny cell layer (Non-Patent Literature 5). An increase in glycerol content of the horny cell layer can be expected to produce a preventing effect on evaporation of moisture from the horny cell layer.

Since improvement of skin moisture richness, skin moisturization and relief of skin dryness are each achieved by increasing the water content of the horny cell layer, these effects can be confirmed by the above-described method for measuring the water content of the horny cell layer. Alternatively, the effects can be sensed by a user of the composition according to the first embodiment of the present invention, or confirmed from assessment by a skin tissue professional such as a dermatologist.

Improvement of skin condition, prevention of skin roughness, improvement of skin roughness, retention of skin functions and improvement of skin functions each mean an effect such that a symptom of skin roughness is improved, or a symptom of skin roughness is prevented.

The symptom of skin roughness refers to any one or more selected from the group consisting of redness, itching, dryness, breakouts, excessive sebum secretion or reduced sebum secretion of the skin.

The symptom of skin roughness can be improved by enhancing the moisturizing capacity (barrier functions) or maintaining skin moisture richness.

Improvement of skin condition, prevention of skin roughness, improvement of skin roughness, retention of skin functions and improvement of skin functions can be sensed by a user of the composition according to the first embodiment of the present invention, or confirmed from assessment by a skin tissue professional such as a dermatologist.

Wound healing is promoted by improving the elasticity of skin epithelial tissues of horny cell layers in the vicinity of the wound, and the elasticity of skin epithelial tissues is improved by increasing the water content of the skin epithelial tissues. The effect can be confirmed by observation of a wound healing process progressing at a high rate. The wound healing process refers to a process in which granulation tissues change into scar tissues. The wound healing process progressing at a high rate can be sensed by a user of the composition according to the first embodiment of the present invention, or confirmed from assessment by a skin tissue professional such as a dermatologist.

### (Second Embodiment)

A composition according to the second embodiment of the present invention, which is a composition for promoting expression of aquaporin 3, will now be described. The composition includes a galloyl group-containing flavan-3-ol monomer and/or a galloyl group-containing flavan-3-ol polymer as an active ingredient.

The galloyl group-containing flavan-3-ol monomer and/or the galloyl group-containing flavan-3-ol polymer has, for example, an increasing effect on the expression level of aquaporin 3 in skin epithelial cells. Thus, expression of aquaporin 3 can be promoted by using the composition according to the second embodiment of the present invention, which includes a galloyl group-containing flavan-3-ol monomer and/or a galloyl group-containing flavan-3-ol polymer.

Preferably, the composition according to the second embodiment of the present invention is used for promoting expression of aquaporin 3 in skin epithelial cells.

As used herein, the "expression level of aquaporin 3" means both the expression level of aquaporin 3 as a protein and the expression level of a gene encoding aquaporin 3. Therefore, the expression level of aquaporin 3 can be measured at a protein level or a gene level (e.g. mRNA level).

It has been reported that aquaporin 3 is a water transporter, and when the expression level of aquaporin 3 in skin epithelial cells is increased, absorption of water into the horny cell layer increases, leading to an increase in water content of the horny cell layer (Non-Patent Literature 2).

It is considered that aquaporin 3 can function as a glycerol transporter to increase the glycerol content of the horny cell layer (Non-Patent Literature 5). An increase in glycerol content of the horny cell layer can be expected to produce a preventing effect on evaporation of moisture from the horny cell layer.

The nucleotide sequences and amino acid sequences (ORF) of human aquaporin 3 are known, and its "Genbank Accession No." is as follows.

### mRNA sequence: NM_004925

Aquaporin 3 as a protein can be measured through mass spectrometry, various types of chromatography, various types of immunochemical assays or the like. Among these, detection performed through an immunochemical assay using an anti-aquaporin 3 antibody is preferable.

Specific examples of the immunochemical assay include enzyme linked immunosorbent assays (ELISAs), Western blotting, protein chip assays and flow cytometry.

The gene encoding aquaporin 3 can be measured through Northern blotting, PCR, microarrays, live imaging using a fluorescent probe, or the like.

When the gene encoding aquaporin 3 is measured through PCR, it is preferable to measure the gene through real-time PCR (RT-PCR).

The method for using the composition according to the second embodiment of the present invention is not limited, and the composition may be absorbed into the body by oral ingestion, or applied to the skin by putting it on or the like.

### (Other Embodiments)

The present invention also includes the following aspects.

Use of the galloyl group-containing flavan-3-ol monomer and/or the galloyl group-containing flavan-3-ol polymer for promoting expression of aquaporin 3 in skin epithelial cells is one aspect of the present invention.

A composition according to the present invention, which is used for one or more selected from the group consisting of improvement of skin moisture richness, supply of moisture to the skin, skin moisturization, relief of skin dryness, improvement of skin condition, enhancement of skin barrier functions, prevention of skin roughness, improvement of skin roughness, retention of skin functions, improvement of skin functions or promotion of wound healing, is one aspect of the present invention.

Use of the galloyl group-containing flavan-3-ol monomer and/or the galloyl group-containing flavan-3-ol polymer for improving skin moisture richness by promoting expression of aquaporin 3 is one aspect of the present invention. The above-described use may be therapeutic use, or nontherapeutic use. The above-described use is preferably use for humans.

The galloyl group-containing flavan-3-ol monomer and/or the galloyl group-containing flavan-3-ol polymer, and preferred aspects thereof are as described above.

### EXAMPLES

Hereinafter, the composition according to the present invention will be described in more detail by way of Examples, which should not be construed as limiting the scope of the present invention.

### (Example 1)

### (Method for Producing Grape Extract (Grape-Derived Material)

A grape extract was obtained by concentrating red wine under reduced pressure to distill away ethanol. The OPC concentration of the grape extract was 15,000 ppm.

Here, the extract of a material derived from grape including grape seeds, such as wine, includes a galloyl group-containing flavan-3-ol polymer (procyanidin B2-3'-O-gallate (hereinafter, referred to as EC-ECG), procyanidin B4-3'-O-gallate (hereinafter, referred to as C-ECG) or the like), and a galloyl group-containing flavan-3-ol monomer (epicatechin gallate (hereinafter, referred to as ECG) or the like) (Reference 1: "Comparative flavan-3-ol composition of seeds from different grape varieties" Food Chemistry 53 (1995) 197-201; Reference 2: "PROCYANIDIN DIMERS AND TRIMERS FROM GRAPE SEEDS" Phytochemistry Vol. 30. No. 4, pp 1259-1264 1991; Reference 3: "Application of "Grape Seed Polyphenol" Functionality" NEW FOOD INDUSTRY Vol. 43 No. 11 (2001), Separate Volume).

### (Method for Producing Test Beverage)

With the combination shown in Table 1, the grape extract and other substances were added to and mixed with water to produce a composition (test food) of Example 1.

**[Table 1]**

| Raw material | Proportion of raw material per liter of composition | |
|---|---|---|
| | Example 1 | Comparative Example 1 |
| Grape extract | 68.5 | - |
| Acesulfame potassium | - | 0.225 |
| Sucralose | 0.19 | 0.028 |
| Sucrose | 2.00 | 16.50 |
| Malic acid | 2.00 | 0.44 |
| Citric anhydride | 0.77 | 0.18 |
| Hardly digestible dextrin | - | 3.00 |

### (Comparative Example 1)

With the combination shown in Table 1, the substances were mixed to produce a composition (control food) of Comparative Example 1.

### (Evaluation of Skin Moisture Richness)

A human test was conducted through the following method to evaluate effects of the compositions of Example 1 and Comparative Example 1 on skin moisture richness. This test was conducted while full consideration was given in keeping with the spirit of Helsinki Declaration.

### (Test Design)

This test was conducted on the basis of the placebo-controlled randomized double-blind parallel-group study in a single facility (UMIN Test ID: UMIN000024478).

### (Subjects)

Subjects were 100 healthy women who were 30 years old or over and under 60 years old and who had been diagnosed by principal investigators or sub-investigators as having recognizable solar specks on cheeks.

The subjects were randomized into two groups. One group was designated as a test food ingestion group (50 subjects), and the other group was designated as control food ingestion group (50 subjects).

In analysis for this test, 3 subjects were found to fall under rejection criteria. Thus, these 3 subjects were excluded, and analysis was performed for 97 subjects (50 subjects in test food ingestion group and 47 subjects in control food ingestion group).

### (Method for Ingesting Test Food)

The subjects in the test food ingestion group ingested the test food (composition of Example 1) at any time once a day over 12 weeks.

The ingestion time until the end of ingestion after the start of ingestion of the test food was set to 1 hour or less.

The subjects in the control food ingestion group ingested the control food (composition of Comparative Example 1) at any time once a day over 12 weeks.

The ingestion time until the end of ingestion after the start of ingestion of the control food was set to 1 hour or less.

### (Measurement of Water Content of Horny Cell Layer)

At 0 week, 4 weeks, 8 weeks and 12 weeks, the water content (AU: arbitrary unit) of the horny cell layer of the subject was measured.

A subject who had finished cleansing away a cosmetic and washing the face was guided into a constant temperature and humidity room (temperature: 21±1°C, humidity 50±5%), and acclimatized at rest for 20 minutes.

Next, a portion which is a point of intersection between the tail of the eye and the nostril was designated as a measurement portion, and measurements were made at the same position. Next, the water content of the horny cell layer at the above-described portion (the same position) was measured five times with Corneometer CM825 (manufactured by Courage + Khazaka electronic GmbH).

Among the obtained values, the highest value and the lowest value were excluded, and an average of the other three measured values was defined as the "water content of the horny cell layer".

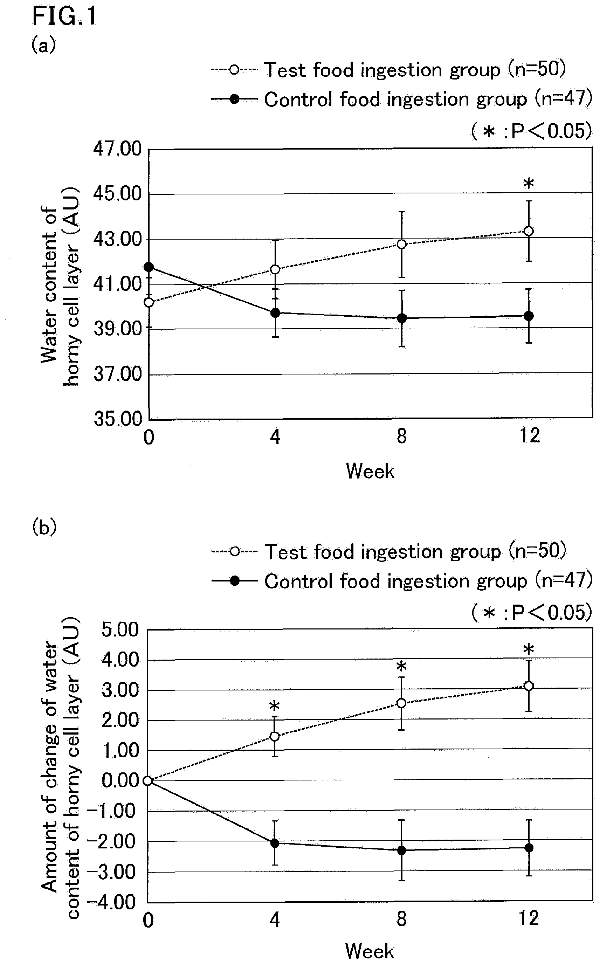

FIGS. 1(a) and 1(b) and Table 2 show the results.

FIG. 1(a) is a graph showing the water contents of horny cell layers of subjects in an evaluation test for skin moisture richness. FIG. 1(b) is a graph showing the amounts of change of the water contents of horny cell layers of subjects in the evaluation test for skin moisture richness.

In this test, statistical analysis was performed with IBM SPSS Statistics 24, a t-test was employed for the statistical significant difference test, and the significance level was set to 5% (on both sides).

The error bar in FIGS. 1(a) and 1(b) represents a standard error (SE), and the symbol "*" indicates that the p-value is less than 0.05 (p < 0.05).

**[Table 2]**

| | Group | 0week | | | | 4 weeks | | | | 8 weeks | | | | 12 weeks | | | |
|---|---|---|---|---|---|---|---|---|---|---|---|---|---|---|---|---|---|
| | | Average | ±SE | t-test 1 | t-test 2 | Average | ±SE | t-test 1 | t-test 2 | Average | ±SE | t-test 1 | t-test 2 | Average | ±SE | t-test 1 | t-test 2 |
| Water content of horny cell layer | Test food ingestion group (n = 50) | 40.21 | ±1.10 | 0.344 | - | 41.65 | ±1.29 | 0.255 | 0.033 | 42.73 | ±1.46 | 0.093 | 0.006 | 43.28 | ±1.34 | 0.040 | 0.001 |
| | Control food ingestion group (n = 47) | 41.78 | ±1.23 | | - | 39.72 | ±1.08 | | 0.007 | 39.44 | ±1.25 | | 0.023 | 39.51 | ±1.20 | | 0.017 |
| Amount of change of water content of horny cell layer at 0 week | Test food ingestion group (n = 50) | - | - | - | - | 1.45 | ±0.66 | 0.001 | - | 2.52 | ±0.88 | 0.000 | - | 3.07 | ±0.85 | 0.000 | - |
| | Control food ingestion group (n = 47) | - | - | | - | -2.06 | ±0.72 | | - | | -2.33 ±0.99 | | - | -2.27 | ±0.92 | | - |

| | | | | | | | | | | | | | | | | | |
|---|---|---|---|---|---|---|---|---|---|---|---|---|---|---|---|---|---|
| t-test 1: unpaired t-test 1 (vs Control food ingestion group) t-test 2: paird t-test 1 (vs 0 week) | | | | | | | | | | | | | | | | | |

12 weeks after the ingestion, the test food ingestion group had a significantly larger water content of the horny cell layer as compared to the control food ingestion group, and analysis with the amount of change showed that 4, 8 and 12 weeks after the ingestion, the test food ingestion group had a significantly larger water content of the horny cell layer as compared to the control food ingestion group.

The water content of the horny cell layer in the test food ingestion group at 4 weeks, 8 weeks and 12 weeks was significantly larger than the water content of the horny cell layer in the test food ingestion group at 0 week.

### (Examples 2-1 to 2-8)

The wines, whose species and production areas are shown in Table 3, were designated as compositions of Examples 2-1 to 2-8.

**[Table 3]**

| | Species | Production area | Total polyphenol | OPC |
|---|---|---|---|---|
| | | | (mg/L) | (mg/L) |
| Example 2-1 | Merlot | Argentine | 3431 | 1576 |
| Example 2-2 | Merlot | Chile | 2171 | 525 |
| Example 2-3 | Merlot | Chile | 2573 | 1506 |
| Example 2-4 | Merlot | Macedonia | 3453 | 703 |
| Example 2-5 | Garnacha Tintorera | Chile | 3320 | 825 |
| Example 2-6 | Ancellotta | Italy | 7157 | 3033 |
| Example 2-7 | Monastrell | Spain | 2672 | 1056 |
| Example 2-8 | Tempranillo | Spain | 2544 | 1392 |

### (Measurement of Total Polyphenol and OPC)

For the compositions of Examples 2-1 and 2-8, the amount of total polyphenol was measured through the Folin-Ciocalteu method, and the amount of OPC was measured through the method described in JP 4659407 B.

Table 3 shows the results.

As described above, the wine includes a galloyl group-containing flavan-3-ol polymer (EC-ECG, C-ECG or the like) and a galloyl group-containing flavan-3-ol monomer (ECG) .

The compositions of Examples 2-1 to 2-8 were applied to skin epithelial cells through the following method, and the expression levels of aquaporin 3 were measured.

### (Method for Evaluating Expression Level of Aquaporin 3)

### (1) Preparation of test sample

The compositions of Examples 2-1 to 2-8 were freeze-dried, and then dissolved in 50% ethanol to prepare test samples.

As a control, 50% ethanol was prepared.

As a positive control, retinoic acid was prepared. The retinoic acid is known as a substance which increases the expression level of aquaporin 3 in skin epithelial cells.

### (2) Culture of Skin Epithelial Cells

Human epidermal keratinocytes (NHEK (AD)) were precultured under conditions of 37°C and 5% CO₂ in a serum-free liquid medium for growing human epidermal keratinocyte (HuMedia-KG2 manufactured by Kurabo Industries, Ltd.) in a 21 cm² dish, and cultured until the cells became sub-confluent.

Next, each test sample was added to the culture solution. Each test sample was added to the culture solution in such a manner that the concentration of the wine in the culture solution was equivalent to 1/400 of the concentration of each original composition (wine).

After each test sample was added, the cells were cultured under conditions of 37°C and 5% CO₂ for 24 hours.

Instead of each test sample, 0.125 mL of 50% ethanol as a control was added to 5 mL of the culture solution.

Instead of each test sample, retinoic acid as a positive control was added to the culture solution in such a manner that the concentration of the retinoic acid in the culture solution was 2 µM.

After the compositions as the controls were added, the cells were cultured under conditions of 37°C and 5% CO₂ for 24 hours.

### (3) Measurement of Expression Level of Aquaporin 3

After the culture of skin epithelial cells in "(2) Culture of Skin Epithelial Cells", RNA was extracted from the cultured cells with ISOGEN (manufactured by Nippon Gene Co., Ltd.).

From 1 µg of the resulting RNA, cDNA was synthesized with Superscript VILO Master Mix (manufactured by Invitrogen).

Thereafter, with primers (primers having nucleotide sequences of SEQ ID NOS: 1 and 2) shown in Table 4, real-time PCR was performed to detect expression of aquaporin 3 (Genbank Accession No. NM_004925).

For PCR, KOD SYBR qPCR Mix (manufactured by Toyobo Co., Ltd.) was used, and the reaction conditions were set such that thermal denaturation was carried out at 98°C for 10 seconds, annealing was carried out at 60°C for 10 seconds, and elongation reaction was carried out at 68°C for 30 seconds.

The fluorescence intensity in the process of amplification was monitored with a quantitative PCR apparatus StepOnePlus (manufactured by Applied Biosystems) to determine the number of cycles taken until the fluorescence intensity became constant. From the number of cycles for aquaporin 3, the expression level was calculated through a standard method.

In parallel to detection of the expression level of aquaporin 3 by real-time PCR, the expression level of glyceraldehyde-3-phosphate dehydrogenase (GAPDH) (Genbank Accession No. NM_002046) which is a housekeeping gene was measured with primers (primers having nucleotide sequences of SEQ ID NOS: 3 and 4) shown in Table 4.

**[Table 4]**

| | | |
|---|---|---|
| Primer for aquaporin 3 | Forward | CTGGGGACCCTCATCCTGGTGATG |
| | Reverse | CACGATAAGGGAGGCTGTGCCTATG |
| Primer for GAPDH | Forward | ACTGGTGTCTTCACCACCATGGAGAAGGC |
| | Reverse | CATGAGGTCCACCACCCTGTTGCTGTAGC |

The expression level of aquaporin 3 in the cultured cells was corrected for the expression level of GAPDH, and the expression level relative against the expression level of aquaporin 3 in the control was calculated, where the expression level of aquaporin 3 in the control was defined as 1.0.

In this test, the minimum number of samples was 3.

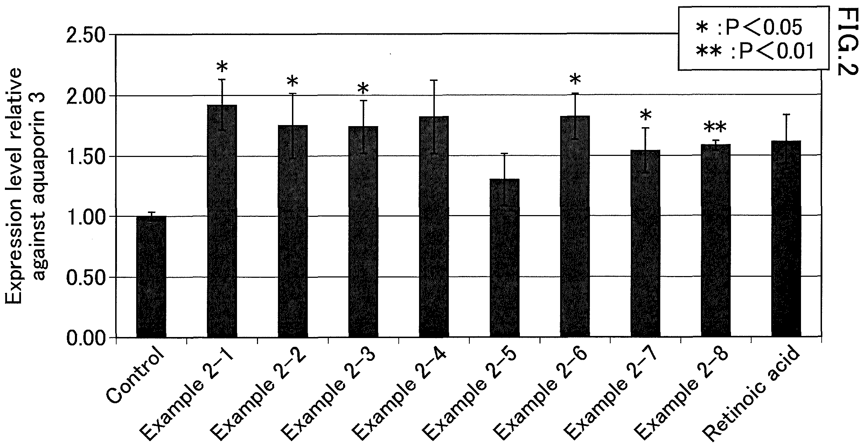

FIG. 2 and Table 5 show the results.

FIG. 2 is a graph showing the results of measuring the expression levels of aquaporin 3 when the compositions of Examples 2-1 to 2-8 in the present invention were used.

In this test, statistical analysis was performed with Excel, a t-test was employed for the statistical significant difference test, and the significance level was set to 5% (on both sides).

The error bar in FIG. 2 represents a standard error (SE), the symbol "*" indicates that the p-value is less than 0.05 (p < 0.05), and the symbol "**" indicates that the p-value is less than 0.01 (p < 0.01).

**[Table 5]**

| | Control | Example 2-1 | Example 2-2 | Example 2-3 | Example 2-4 | Example 2-5 | Example 2-6 | Example 2-7 | Example 2-8 | Retinoic acid |
|---|---|---|---|---|---|---|---|---|---|---|
| Relative gene expression level (average) | 1.00 | 1.92 | 1.75 | 1.74 | 1.82 | 1.30 | 1.82 | 1.54 | 1.58 | 1.61 |
| SE | 0.03 | 0.21 | 0.27 | 0.22 | 0.30 | 0.22 | 0.19 | 0.18 | 0.04 | 0.22 |
| t-test (vs control) | - | 0.011 | 0.038 | 0.026 | 0.052 | 0.238 | 0.011 | 0.097 | 0.00005 | 0.070 |

As shown in FIG. 2 and Table 5, use of the compositions of Examples 2-1 to 2-8 led to an increase in expression level of aquaporin 3 in skin epithelial cells.

### (Separation of Composition of Example)

In order to identify a compound which increases the expression level of aquaporin 3, the composition of Example 2-6 was subjected to separation through the following method.

First, 12 L of the composition of Example 2-6 was concentrated under reduced pressure to distill away ethanol.

Thereafter, the composition from which ethanol had been distilled away was loaded into a 10 L HP-2MG column (manufactured by Supelco, USA) equilibrated with water, and was rinsed with 20 L of distilled water, and then eluted with 30 L of 70% ethanol.

The 70% ethanol-eluted fraction was concentrated under reduced pressure, and freeze-dried.

500 mg of the resulting dried product was loaded into a Sephadex LH-20 (1L, GE healthcare) column, and eluted with 4 L of each of 1% formic acid-containing 10%, 20%, 30%, 40%, 50% and 60% methanol/water to be separated into six fractions. A fraction separated with 50% methanol had a galloyl group-containing flavan-3-ol dimer, and a fraction separated with 60% methanol had a galloyl group-containing flavan-3-ol trimer.

The expression level of aquaporin 3 in skin epithelial cells was measured in the same manner as in "Method for Evaluating Expression Level of Aquaporin 3" except that instead of each test sample, 0.0125 mL of each fraction was added to 5 mL of a culture solution.

In this test, the number of samples was 3.

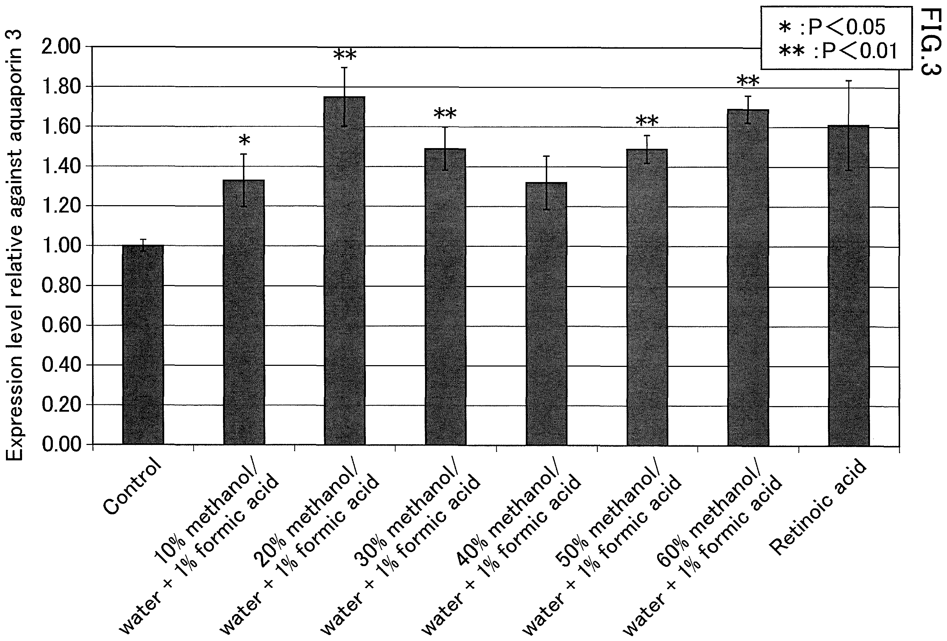

FIG. 3 and Table 6 show the results.

FIG. 3 is a graph showing the results of measuring the expression levels of aquaporin 3 when the fraction of the composition of Example 2-6 was used.

In this test, statistical analysis was performed with Excel, a t-test was employed for the statistical significant difference test, and the significance level was set to 5% (on both sides).

The error bar in FIG. 3 represents a standard error (SE), the symbol "*" indicates that the p-value is less than 0.05 (p < 0.05), and the symbol "**" indicates that the p-value is less than 0.01 (p < 0.01).

**[Table 6]**

| Fraction of composition of Example 2-6 | Control | 10% methanol/water + 1% formic acid | 20% methanol/water + 1% formic acid | 30% methanol/water + 1% formic acid | 40% methanol/water + 1% formic acid | 50% methanol/water + 1% formic acid | 60% methanol/water + 1% formic acid | Retinoic acid |
|---|---|---|---|---|---|---|---|---|
| Relative gene expression level (average) | 1.00 | 1.33 | 1.75 | 1.49 | 1.32 | 1.49 | 1.69 | 1.61 |
| SE | 0.03 | 0.13 | 0.15 | 0.11 | 0.14 | 0.07 | 0.07 | 0.22 |
| t-test (vs control) | - | 0.042 | 0.001 | 0.005 | 0.065 | 0.0004 | 0.000004 | 0.070 |

As shown in FIG. 3 and Table 6, it was indicated that a compound having increasing action on the expression level of aquaporin 3 in skin epithelial cells was present in a large amount in a fraction separated with 20% methanol/water and a fraction separated with 60% methanol/water.

### <Production Example 1>

Flavan-3-ol monomers and/or polymers to be used in Examples and Comparative Examples were prepared through the following method.

As EC-ECG and EC-CG, those obtained by performing synthesis in accordance with the literature (Food chem., 2018, Takahiro Fujimaki et. al 248, P61-69) were used.

Procyanidin B3 (hereinafter, referred to as C-C), procyanidin B4 (hereinafter, referred to as C-EC), C-ECG and C-C-ECG were synthesized in the following manner in accordance with the literature (Food chem., 2018, Takahiro Fujimaki et. al 248, P61-69).

C-C: 500 mg of (+)-taxifolin (manufactured by Extrasynthese) in 50 ml of ethanol, 200 mg of NaBH₄ was added, and the resulting mixture was stirred at room temperature for 45 minutes. In the resulting solution, 1 g of (+)-catechin (C) (manufactured by Sigma-Aldrich Co. LLC.) was dissolved, 60 ml of HCl (0.1 N) was then added, and the resulting mixture was stirred for 1 hour. The reaction product was purified by reverse-phase HPLC to give 200 mg of C-C and 1 mg of a C-C-C trimer.

C-EC: 500 mg of (+)-taxifolin was dissolved in 50 ml of ethanol, 200 mg of NaBH₄ was added, and the resulting mixture was stirred at room temperature for 45 minutes. In the resulting solution, 1 g of (-)-epicatechin (EC) (manufactured by Sigma-Aldrich Co. LLC.) was dissolved, 60 ml of HCl (0.1 N) was then added, and the resulting mixture was stirred for 1 hour. The reaction product was purified by reverse-phase HPLC to give 200 mg of C-EC.

C-ECG and C-C-ECG: 350 mg (1.15 mmol) of (+)-taxifolin was dissolved in 20 ml of ethanol, 70 mg of NaBH₄ was added, and the resulting mixture was stirred at room temperature for 45 minutes. In the resulting solution, 420 mg (0.95 mmol) of (-)-epicatechin-3-O-gallate (ECG) (manufactured by FUJIFILM Wako Pure Chemical Corporation) was dissolved, 40 ml of HCl (0.1 N) was then added, and the resulting mixture was stirred for 1 hour. The reaction product was purified by reverse-phase HPLC to give 96 mg of C-ECG and 34 mg of C-C-ECG.

Procyanidin B1 (hereinafter, referred to as EC-C) used was one manufactured by Extrasynthese, procyanidin B2 (hereinafter, referred to as EC-EC) used was one manufactured by Tront Research Chemicals Inc., and procyanidin C1 (PC1) used was one manufactured by Sigma-Aldrich Co. LLC.

### (Reference Examples 1 and 2, Examples 3-1 to 3-4 and Comparative Examples 2-1 to 2-4)

The aquaporin 3 expression promoting activity was measured for catechin (C) (Comparative Examples 2-1 and 2-2), epicatechin (EC) (Comparative Examples 2-3 and 2-4), epicatechin gallate (ECG) (Examples 3-1 and 3-2) and epigallocatechin gallate (EGCG) (Examples 3-3 and 3-4).

EGCG used was one manufactured by FUJIFILM Wako Pure Chemical Corporation.

A cell culture solution was used as a control in Reference Example 1, and retinoic acid (RA) (manufactured by Sigma-Aldrich Co. LLC.) was used as Reference Example 2 (positive control).

### (Method for Evaluating Expression Level of Aquaporin 3)

Each compound was dissolved in a human epidermal keratinocyte growing serum-free liquid medium (HuMedia-KG2 manufactured by Kurabo Industries, Ltd.) to prepare a test sample. Except for the above, the same procedure as in Examples 2-1 and 2-2 was carried out to evaluate the expression level of aquaporin 3 in each of Reference Examples 1 and 2, Examples 3-1 to 3-4 and Comparative Examples 2-1 to 2-4.

In Examples 3-1 to 3-4 and Comparative Examples 2-1 to 2-4, each test sample was added to the culture solution in such a manner that the concentration of the compound in the culture solution was 30 µM or 50 µM. The culture solution was used as a control.

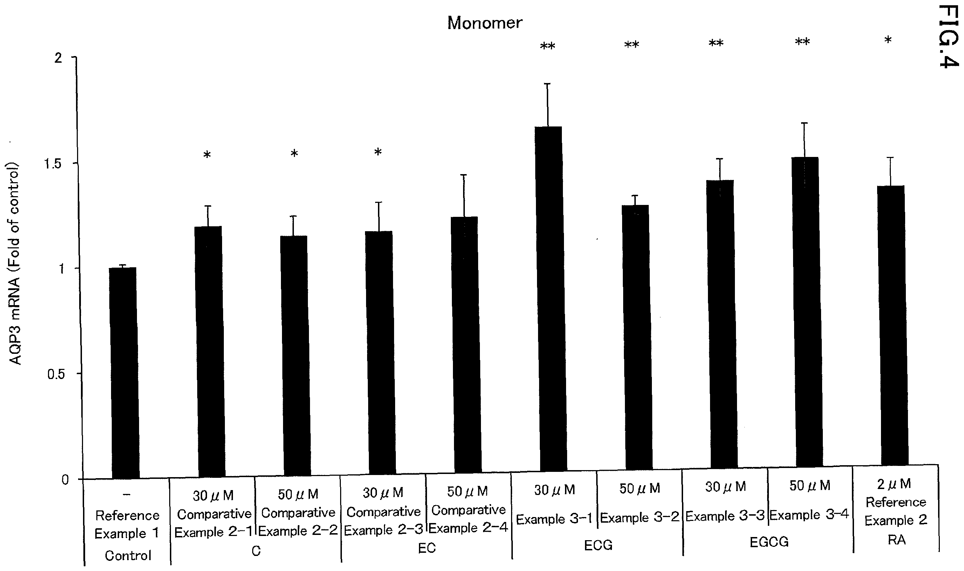

Table 7 and FIG. 4 show the results.

FIG. 4 is a graph showing the results of measuring the expression levels of aquaporin 3 when the compositions of Reference Examples 1 and 2, Examples 3-1 to 3-4 and Comparative Examples 2-1 to 2-4 were used.

In this test, statistical analysis was performed with Excel, a t-test was employed for the statistical significant difference test, and the significance level was set to 5% (on both sides).

The error bar in FIG. 4 represents a standard error (SE), the symbol "*" indicates that the p-value is less than 0.05 (p < 0.05), and the symbol "**" indicates that the p-value is less than 0.01 (p < 0.01).

**[Table 7]**

| | Control | C | | EC | | ECG | | EGCG | | RA |
|---|---|---|---|---|---|---|---|---|---|---|
| | Reference Example 1 | Comparative Example 2-1 | Comparative Example 2-2 | Comparative Example 2-3 | Comparative Example 2-4 | Example 3-1 | Example 3-2 | Example 3-3 | Example 3-4 | Reference Example 2 |
| | - | 30 µM | 50 µM | 30 µM | 50 µM | 30 µM | 50 µM | 30 µM | 50 µM | 2 µM |
| Relative gene expression level (average) | 1.00 | 1.19 | 1.14 | 1.15 | 1.21 | 1.63 | 1.25 | 1.36 | 1.47 | 1.32 |
| SE | 0.01 | 0.10 | 0.09 | 0.14 | 0.20 | 0.20 | 0.05 | 0.10 | 0.16 | 0.13 |
| t-test (vs control) | - | 0.001 | 0.011 | 0.037 | 0.055 | 0.000 | 0.000 | 0.000 | 0.000 | 0.003 |

### (Reference Examples 3 and 4, Examples 4-1 to 4-4 and Comparative Examples 3-1 to 3-8)

Except that instead of flavan-3-ol monomers such as catechin, EC-C (Comparative Examples 3-1 and 3-2), EC-EC (Comparative Examples 3-3 and 3-4), C-C (Comparative Examples 3-5 and 3-6) and C-EC (Comparative Examples 3-7 and 3-8) shown in Table 8 were used, the same procedure as in Comparative Examples 2-1 to 2-4 was carried out to measure the aquaporin 3 expression promoting activity.

Except that C-ECG (PB4-GA) (Examples 4-1 and 4-2) and EC-CG (Examples 4-3 and 4-4) shown in Table 8 were used as galloyl group-containing flavan-3-ol dimers, the same procedure as in Comparative Examples 3-1 to 3-4 was carried out to measure the aquaporin 3 expression promoting activity.

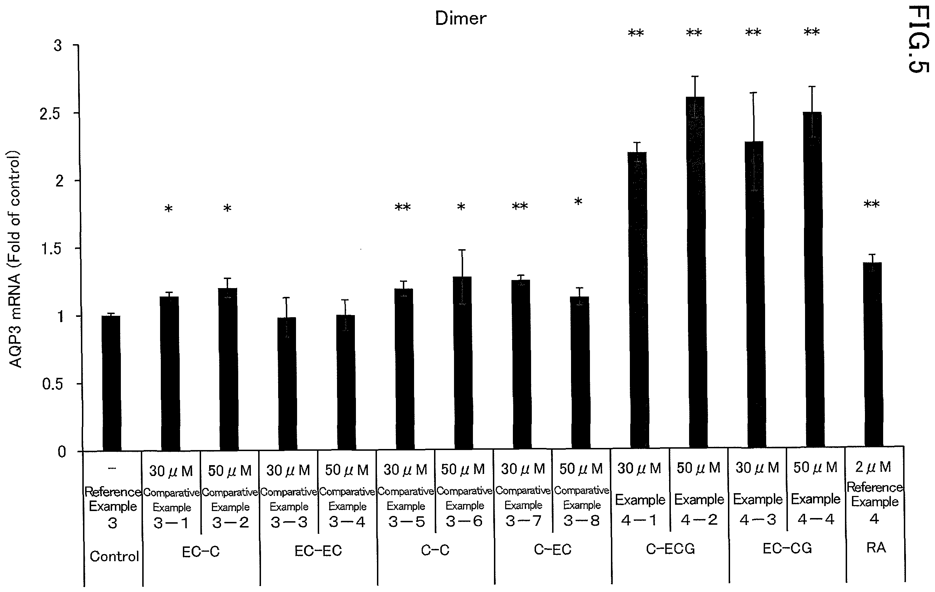

A cell culture solution was used as a control in Reference Example 3, and retinoic acid (RA) (manufactured by Sigma-Aldrich Co. LLC.) was used as Reference Example 4 (positive control). Table 8 and FIG. 5 show the results.

**[Table 8]**

| | Conrtol | EC-C | | EC-EC | | C-C | | C-EC | | C-ECG | | EC-CG | | RA |
|---|---|---|---|---|---|---|---|---|---|---|---|---|---|---|
| | Reference Example 3 | Comparative Example 3-1 | Comparative Example 3-2 | Comparative Example 3-3 | Comparative Example 3-4 | Comparative Example 3-5 | Comparative Example 3-6 | Comparative Example 3-7 | Comparative Example 3-8 | Example 4-1 | Example 4-2 | Example 4-3 | Example 4-4 | Reference Example 4 |
| | - | 30 µM | 50 µM | 30 µM | 50 µM | 30 µM | 50 µM | 30 µM | 50 µM | 30 µM | 50 µM | 30 µM | 50 µM | 2 µM |
| Relative gene expression level (average) | 1.00 | 1.14 | 1.20 | 0.98 | 0.99 | 1.19 | 1.27 | 1.24 | 1.12 | 2.19 | 2.59 | 2.26 | 2.47 | 1.36 |
| SE | 0.02 | 0.03 | 0.07 | 0.15 | 0.11 | 0.05 | 0.20 | 0.03 | 0.06 | 0.07 | 0.15 | 0.36 | 0.19 | 0.06 |
| t-test (vs control) | - | 0.007 | 0.001 | 0.767 | 0.927 | 0.001 | 0.016 | 0.000 | 0.023 | 0.000 | 0.000 | 0.000 | 0.000 | 0.000 |

### (Reference Examples 5 and 6, Examples 5-1 and 5-2 and Comparative Examples 4-1 to 4-4)

Except that instead of flavan-3-ol monomers such as catechin, flavan-3-ol trimers (EC-EC-EC (PC1) (Comparative Examples 4-1 and 4-2) and C-C-C (Comparative Examples 4-3 and 4-4)) shown in Table 9 were used as Comparative Examples, the same procedure as in Comparative Examples 2-1 to 2-4 was carried out to measure the aquaporin 3 expression promoting activity.

Except that C-C-ECG (Examples 5-1 and 5-2) shown in Table 9 was used as a galloyl group-containing flavan-3-ol trimer, the same procedure as in Comparative Examples 3-1 to 3-4 was carried out to measure the aquaporin 3 expression promoting activity.

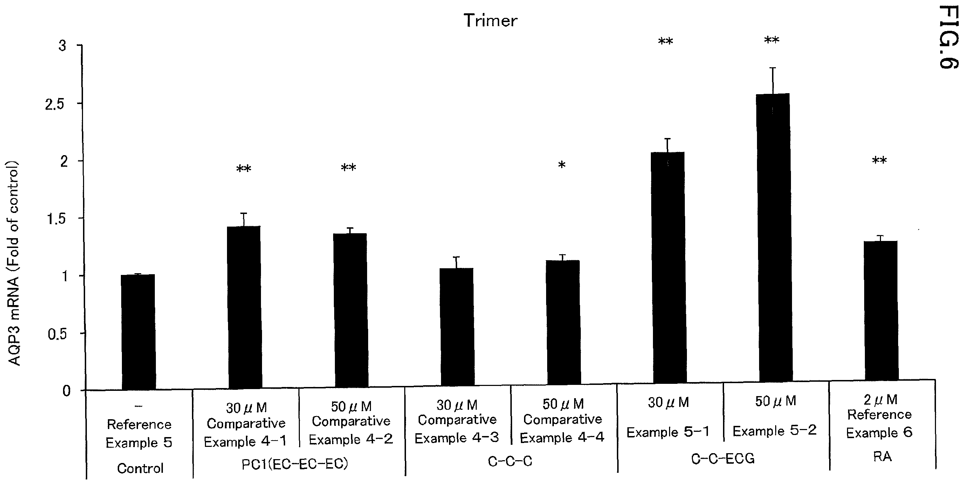

A cell culture solution was used as a control in Reference Example 5, and retinoic acid (RA) (manufactured by Sigma-Aldrich Co. LLC.) was used as Reference Example 6 (positive control). Table 9 and FIG. 6 show the results.

**[Table 9]**

| | Contrl | PC1 (EC-EC-EC) | | C-C-C | | C-C-ECG | | RA |
|---|---|---|---|---|---|---|---|---|
| | Reference Example 5 | Comparative Example 4-1 | Comparative Exampl e4-2 | Comparative Example 4-3 | Comparative Example 4-4 | Example 5-1 | Example 5-2 | Reference Example 6 |
| | - | 30 µM | 50 µM | 30 µM | 50 µM | 30 µM | 50 µM | 2 µM |
| Relative gene expression level (average) | 1.00 | 1.41 | 1.34 | 1.02 | 1.08 | 2.01 | 2.50 | 1.21 |
| SE | 0.01 | 0.11 | 0.05 | 0.10 | 0.05 | 0.12 | 0.23 | 0.05 |
| t-test (vs control) | - | 0.000 | 0.000 | 0.706 | 0.029 | 0.000 | 0.000 | 0.000 |

The results in Tables 6 to 9 and FIGS. 4 to 6 show that flavan-3-ol monomers and dimeric or higher order flavan-3-ol polymers with a galloyl group (that is, galloyl group-containing flavan-3-ol monomers and galloyl group- containing flavan-3-ol polymers) have an increasing effect on expression of aquaporin 3.

### INDUSTRIAL APPLICABILITY

The composition of the present invention can be used for improving skin moisture richness and increasing the expression level of aquaporin 3 in skin epithelial cells.

## Claims

1. A composition for promoting expression of aquaporin 3, comprising a galloyl group-containing flavan-3-ol monomer and/or a galloyl group-containing flavan-3-ol polymer as an active ingredient.

2. The composition according to claim 1,
wherein the galloyl group-containing flavan-3-ol monomer and/or the galloyl group-containing flavan-3-ol polymer is present in a grape-derived material.

3. The composition according to claim 2,
wherein the grape-derived material contains at least one selected from the group consisting of at least one raw material selected from the group consisting of a pericarp, a fruit or a seed of grape; a fermented product of the raw material; an extract thereof; or a processed product thereof.

4. The composition according to any one of claims 1 to 3,
wherein the galloyl group-containing flavan-3-ol monomer is a compound represented by the following formula (1) and/or a compound represented by the following formula (2) .

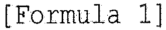

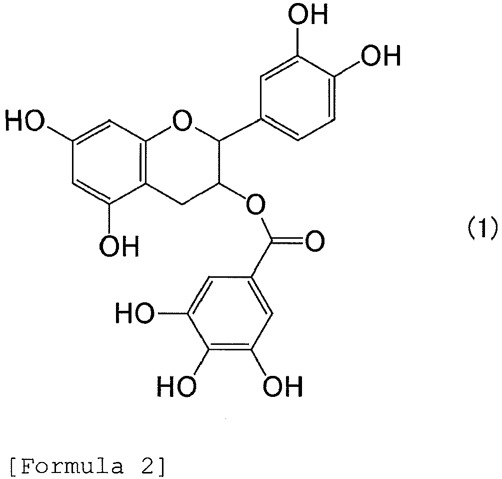

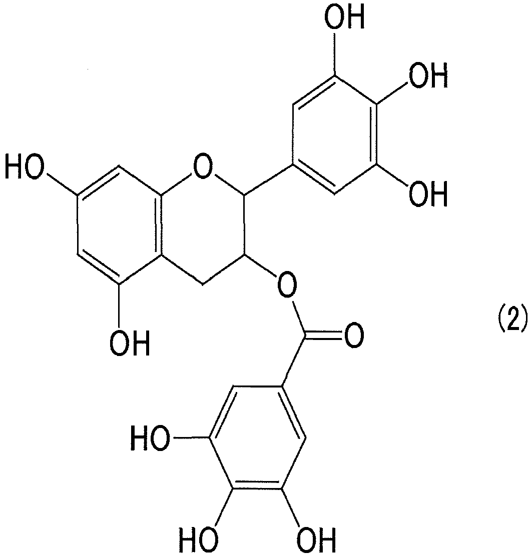

5. The composition according to any one of claims 1 to 4,
wherein the galloyl group-containing flavan-3-ol polymer is a compound represented by the following formula (3) and/or a compound represented by the following formula (4) .

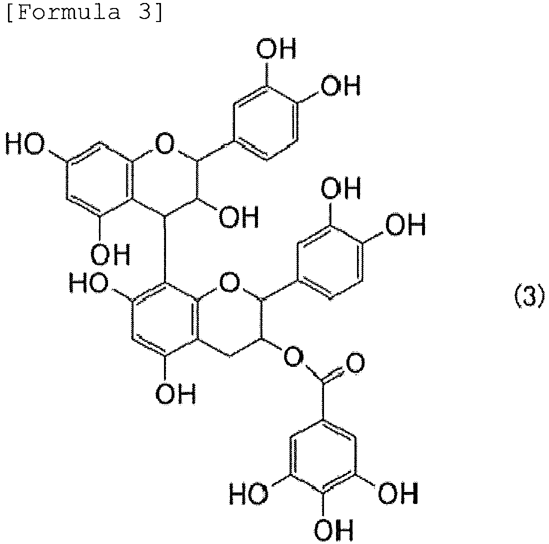

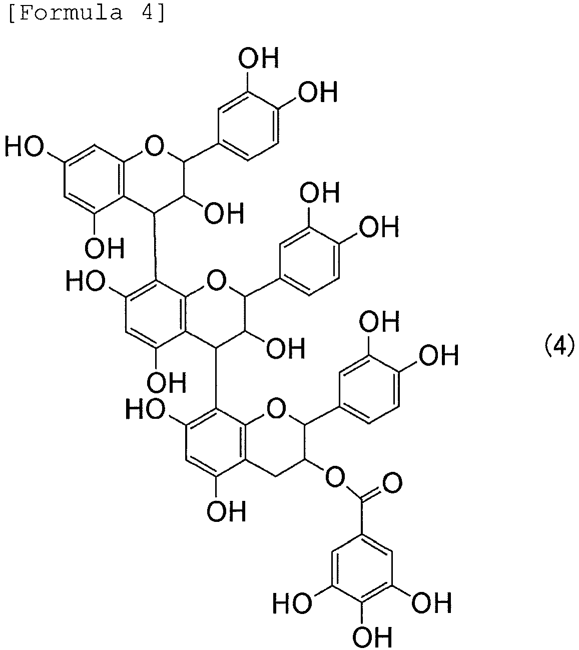

6. The composition according to any one of claims 1 to 5,
wherein the composition is a food or beverage product, a cosmetic, a pharmaceutical product or a quasi-pharmaceutical product.

7. The composition according to any one of claims 1 to 6,
wherein the composition is used for one or more selected from the group consisting of improvement of skin moisture richness, supply of moisture to the skin, retention of skin moisture, skin moisturization, relief of skin dryness, improvement of skin condition, enhancement of skin barrier functions, prevention of skin roughness, improvement of skin roughness, retention of skin functions, improvement of skin functions or promotion of wound healing.

8. Use of a galloyl group-containing flavan-3-ol monomer and/or a galloyl group-containing flavan-3-ol polymer for promoting expression of aquaporin 3.

9. Use of a galloyl group-containing flavan-3-ol monomer and/or a galloyl group-containing flavan-3-ol polymer for improving skin moisture richness by promoting expression of aquaporin 3.


### SEQUENCE LISTING

SD60WO Sequence Listing.txt

SEQUENCE LISTING

<110>
\203T\203\223\203g\203\212\201[\203z\201[\203\213\203f\203B\203\223\203O\203X
\212\224\216®\211ï\216Ð\201@Suntory Holdings Limited

<120>
\203A\203N\203A\203|\203\212\203\223\202R\224-\214»\221£\220i\227p\221g\220¬
\225¨\213y\202Ñ\202»\202Ì\216g\227p

<130>  SP2018-0084

<150>  JP2017-191860
<151>  2017-09-29

<160>  4

<170>  PatentIn version 3.5

<210>  1
<211>  24
<212>  DNA
<213>  Artificial Sequence

<220>
<223>  hAQP3 Forward primer

<400>  1
ctggggaccc tcatcctggt gatg                                              24


<210>  2
<211>  25
<212>  DNA
<213>  Artificial Sequence

<220>
<223>  hAQP3 Reverse primer

<400>  2
cacgataagg gaggctgtgc ctatg                                             25


<210>  3
<211>  29
<212>  DNA
<213>  Artificial Sequence

<220>
<223>  hGAPDH Forward primer

<400>  3
actggtgtct tcaccaccat ggagaaggc                                         29


<210>  4
<211>  29
<212>  DNA
<213>  Artificial Sequence

<220>
<223>  hGAPDH Reverse primer

<400>  4
catgaggtcc accaccctgt tgctgtagc                                          29

SEQUENCE LISTING

<110>  Suntory Holdings Limited

<120>  COMPOSITION FOR PROMOTING EXPRESSION OF AQUAPORIN 3, AND USE THEREOF

<130>  AD1549 EP

<140>  EP 18 86 1419.2
<141>  2018-09-28

<150>  JP2017-191860
<151>  2017-09-29

<160>  4

<170>  PatentIn version 3.5

<210>  1
<211>  24
<212>  DNA
<213>  Artificial Sequence

<220>
<223>  hAQP3 Forward primer

<400>  1
ctggggaccc tcatcctggt gatg                                              24


<210>  2
<211>  25
<212>  DNA
<213>  Artificial Sequence

<220>
<223>  hAQP3 Reverse primer

<400>  2
cacgataagg gaggctgtgc ctatg                                             25


<210>  3
<211>  29
<212>  DNA
<213>  Artificial Sequence

<220>
<223>  hGAPDH Forward primer

<400>  3
actggtgtct tcaccaccat ggagaaggc                                         29


<210>  4
<211>  29
<212>  DNA
<213>  Artificial Sequence

<220>
<223>  hGAPDH Reverse primer

<400>  4

catgaggtcc accaccctgt tgctgtagc 29